Europäisches Patentamt

European Patent Office

Office européen des brevets

(19)

(11) Veröffentlichungsnummer: **0 093 867**
**B1**

(12) # EUROPÄISCHE PATENTSCHRIFT

(45) Veröffentlichungstag der Patentschrift:
**04.06.86**

(51) Int. Cl.⁴: **A 61 F 2/60**

(21) Anmeldenummer: **83102934.3**

(22) Anmeldetag: **24.03.83**

(54) **Hüftgelenk für ein künstliches Bein.**

(30) Priorität: **21.04.82 DE 3214773**

(43) Veröffentlichungstag der Anmeldung:
**16.11.83 Patentblatt 83/46**

(45) Bekanntmachung des Hinweises auf die Patenterteilung:
**04.06.86 Patentblatt 86/23**

(84) Benannte Vertragsstaaten:
**AT BE FR GB IT NL SE**

(56) Entgegenhaltungen:
**DE - A - 2 754 384**
**DE - B - 1 922 620**

(73) Patentinhaber: **Otto Bock Orthopädische Industrie Besitz- und Verwaltungs-Kommanditgesellschaft, Industriestrasse, D-3428 Duderstadt (DE)**

(72) Erfinder: **Glabiszewski, Richard, Im Puttfeld 1, D-3428 Duderstadt 15 (DE)**

(74) Vertreter: **Lins, Edgar, Dipl.-Phys. et al, Patentanwälte Gramm + Lins Theodor-Heuss-Strasse 2, D-3300 Braunschweig (DE)**

EP 0 093 867 B1

ACTORUM AG

## Beschreibung

Die Erfindung betrifft ein Hüftgelenk für ein künstliches Bein mit einem aus einem im wesentlichen horizontalen Schenkel und einem im stumpfen Winkel dazu stehenden aufrechten Schenkel bestehenden Befestigungswinkel, an dem ein erstes Gelenkteil befestigt ist, das über ein Drehgelenk mit einem zweiten, zur Verbindung mit dem künstlichen Bein vorgesehenen Gelenkteil verbunden ist, wobei die Drehbewegung des zweiten Gelenkteils durch Anschläge für die stehende und die sitzende Stellung des künstlichen Beins beschränkt ist und zwischen dem ersten und dem zweiten Gelenkteil elastische Mittel eingesetzt sind, die für die Bewegung des zweiten Gelenkteils aus der stehenden in die sitzende Stellung und umgekehrt eine elastische Gegenkraft erzeugen.

Derartige Hüftgelenke werden seit vielen Jahren dazu benutzt, ein vollständiges künstliches Bein beweglich an dem Körper des Beinamputierten anzubringen. Der Befestigungswinkel wird dabei im allgemeinen mit einem um den Unterkörper des Beinamputierten geschnallten Befestigungsteil verbunden. Die elastischen Mittel zwischen dem ersten und zweiten Gelenkteil haben die Aufgabe, das künstliche Bein sicher in der stehenden bzw. sitzenden Stellung zu halten und eine möglichst natürliche Bewegung des künstlichen Oberschenkels zu ermöglichen.

Bei den bekannten Hüftgelenken dieser Art ist das erste Gelenkteil an dem im wesentlichen horizontal stehenden Schenkel des Befestigungswinkels angebracht. Die elastischen Mittel zwischen dem ersten und zweiten Gelenkteil werden durch elastische Bänder gebildet, die zwischen den beiden Gelenkteilen oder dem ersten Gelenkteil und dem künstlichen Bein gespannt sind.

Die bekannten Hüftgelenke haben einen relativ grossen Raumbedarf. Dies führt nicht nur zu kosmetischen Schwierigkeiten, sondern ist insbesondere in der Sitzposition des Prothesenträgers nachteilig. Beim Sitzen stösst nämlich das Hüftgelenk auf die Sitzfläche auf, so dass ein bequemer Sitz erheblich gestört wird. Die Handhabung eines derartigen Hüftgelenks ist daher nicht optimal.

Hüftgelenke älterer Bauart sind mit einer Sperre versehen, die eine Bewegung des Hüftgelenkes nur in einem ganz geringen Masse während des Gehens erlauben. Um die Sitzposition einzunehmen, muss der Prothesenträger die Sperre manuell lösen. Die Sperre erfüllt eine Rastfunktion für die stehende Position. Diese Hüftgelenke sind mit den oben beschriebenen Hüftgelenken nicht vergleichbar, da sie der manuellen Handhabe jederzeit zugänglich sein müssen und die Funktion der Sperre bei den neueren Hüftgelenken durch die elastischen Mittel ausgeübt wird.

Der Erfindung liegt daher die Aufgabe zugrunde, ein Hüftgelenk der eingangs erwähnten Art zu erstellen, das einfacher in der Handhabung und für den Prothesenträger bequemer ist und darüber hinaus die kosmetische Ausgestaltung erleichtert.

Diese Aufgabe wird erfindungsgemäss dadurch gelöst, dass das erste Gelenkteil am aufrechten Schenkel des Befestigungswinkels angebracht ist und dass das erste Gelenkteil und das zweite Gelenkteil in der sitzenden Stellung nicht oder höchstens unwesentlich über die Verlängerungslinie des im wesentlichen horizontalen Schenkels nach unten herausragen.

Das erfindungsgemässe Hüftgelenk weist den wesentlichen Vorteil auf, dass es den Prothesenträger in der sitzenden Stellung überhaupt nicht stört, weil es nach unten eine Bauhöhe Null oder nahezu Null aufweist. Darüber hinaus lässt sich das erfindungsgemässe Hüftgelenk so kompakt bauen, dass es kosmetisch voll verkleidbar ist.

Durch die Anbringung des ersten Gelenkteils an dem aufrechten Schenkel des Befestigungswinkels lässt sich das erste Gelenkteil um einen kleinen Winkel in vertikaler Ebene drehbar an dem Befestigungswinkel anbringen. Dadurch ist es möglich, das künstliche Bein in dem Hüftgelenk in der Frontalebene zu justieren. Während eine derartige Verstellmöglichkeit bei keinem bekannten Hüftgelenk vorgesehen ist, kann bei dem erfindungsgemässen Hüftgelenk eine Anpassung an die individuellen Gegebenheiten des Prothesenträgers erreicht werden, ohne dass hierzu die Beinprothese selbst geändert werden müsste.

In einer einfachen Ausgestaltungsform weist das erste Gelenkteil zwei untereinander angeordnete Löcher auf, durch die die Befestigungsschrauben zur Befestigung an dem Befestigungswinkel ragen und von denen eines als kreisbogenförmiges Langloch ausgebildet ist. Das kreisbogenförmige Langloch ermöglicht dabei die Drehung des ersten Gelenkteils um eine nahezu horizontale Achse, die senkrecht auf dem aufrechten Schenkel des Befestigungswinkels steht.

Zusätzlich zu der Justiermöglichkeit in der Frontalebene wird bei dem erfindungsgemässen Hüftgelenk vorzugsweise eine Justiermöglichkeit in der Sagittalebene dadurch verwirklicht, dass das zweite Gelenkteil in der stehenden Stellung mit seiner Oberseite gegen die Unterseite des ersten Gelenkteils stösst und dass die Höhe der Oberseite des zweiten Gelenkteils einstellbar ist. Durch die Verstellung der Höhe der Oberseite des zweiten Gelenkteils lässt sich der Winkel des Oberschenkels des künstlichen Beins in der stehenden Stellung justieren.

Dies geschieht in einfacher Weise dadurch, dass die Oberseite des zweiten Gelenkteils durch ein keilförmiges Anschlagstück gebildet ist, das auf einer schrägen Fläche eines keilförmigen Gegenstücks aufliegt und dass die relative horizontale Lage der beiden keilförmigen Stücke einstellbar ist. Die Verstellung der horizontalen Lage der beiden keilförmigen Stücke geschieht vorzugsweise dadurch, dass eines der keilförmigen Stücke in horizontaler Richtung ein Durchgangsloch aufweist, durch das eine in einem Gewindeloch des anderen keilförmigen Stücks geführte Schraube hindurchragt, die im Gehäuse des Gelenks in horizontaler Richtung unverschiebbar gelagert ist. Durch die Verstellung der Schraube kann die horizontale relative Position der beiden keilförmigen Stücke verändert werden, wodurch die Höhe der Oberseite des keilförmigen Anschlagstücks veränderbar ist. Zur Geräuschminderung und Dämpfung der Anschlagbewegung ist es vorteilhaft, wenn die Oberseite des zweiten Gelenkteils aus einem elastischen, dämpfenden Material gebildet ist.

Ein wesentlicher Grund für die Unförmigkeit der bekannten Hüftgelenke lag in der Anbringung der elastischen Bänder, die üblicherweise auf beiden Seiten des Hüftgelenks angebracht worden sind. Das erfindungsgemässe Hüftgelenk lässt sich vorzugsweise weiter verkleinern durch einen drehbar am ersten Gelenkteil gelagerten Stift, dessen anderes Ende in dem zweiten Gelenkteil drehbar mit einem Ende einer in einem rohrförmigen Ansatz für ein Rohrskeletteil des künstlichen Beins gelagerten Druckfeder verbunden ist. Erfindungsgemäss lässt sich daher das für die Bewegung des Hüftgelenks erforderliche elastische Mittel ohne zusätzlichen Raumaufwand innerhalb des Gelenks im Rahmen der Rohrskelettbauweise verwirklichen, wobei die Möglichkeit der kosmetischen Verkleidung weiter verbessert wird. Die Vorspannung der Druckfeder ist vorzugsweise einstellbar, um die elastische Kraft den individuellen Gegebenheiten anpassen zu können. Bei den bekannten Hüftgelenken konnte eine derartige Anpassung nur durch Auswechslung der elastischen Bänder erfolgen.

Die drehbare Anlenkung des Stiftes an dem ersten Gelenkteil erfolgt vorzugsweise oberhalb des Anlenkpunktes an der Druckfeder in der stehenden Stellung und unterhalb des Anlenkpunktes der Feder in der sitzenden Stellung. Dadurch wird erreicht, dass über einen gewissen Bewegungswinkelbereich des Hüftgelenks die Federkraft zunimmt, während sie abnimmt, wenn eine gewisse Auslenkung des Hüftgelenks überschritten wird. Diese Auslenkung betrifft vorzugsweise etwa 90°. Ist das Hüftgelenk um diese 90° ausgelenkt worden, sorgt die Feder dafür, dass die weitere Bewegung in die sitzende Stellung des Hüftgelenks durch die Federkraft erleichtert wird.

Die Erfindung soll im folgenden anhand eines in der Zeichnung dargestellten Ausführungsbeispiels näher erläutert werden. Es zeigen:

Fig. 1 eine Schnittdarstellung des Hüftgelenks in stehender Stellung;

Fig. 2 eine Schnittdarstellung des Hüftgelenks in sitzender Stellung;

Fig. 3 eine Schnittdarstellung eines Details zur Anschlagjustierung in der stehenden Stellung;

Fig. 4 eine Vorderansicht eines Details zur Frontaljustierung;

Fig. 5 eine Ansicht eines künstlichen Beins mit Darstellung des Justierbereichs in der Frontalebene;

Fig. 6 eine Seitenansicht des künstlichen Beins mit Darstellung der Justiermöglichkeiten in der Sagittalebene;

Fig. 7 eine Seitenansicht des künstlichen Beins in der sitzenden Stellung.

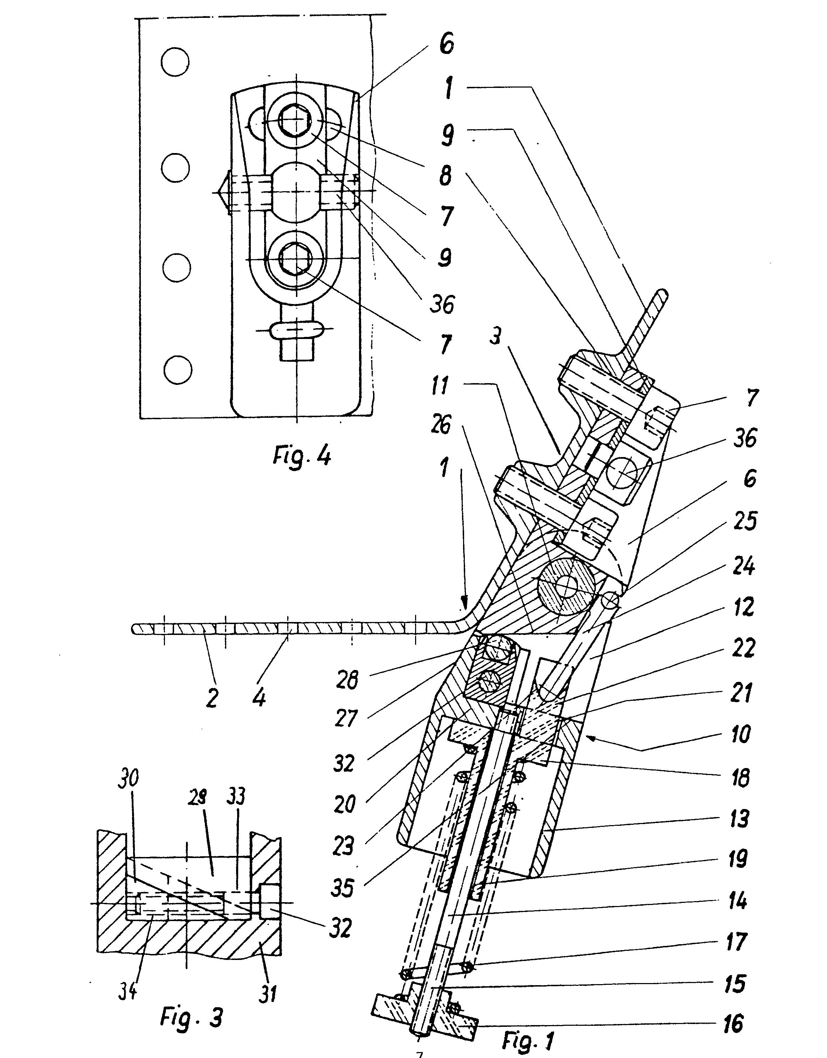

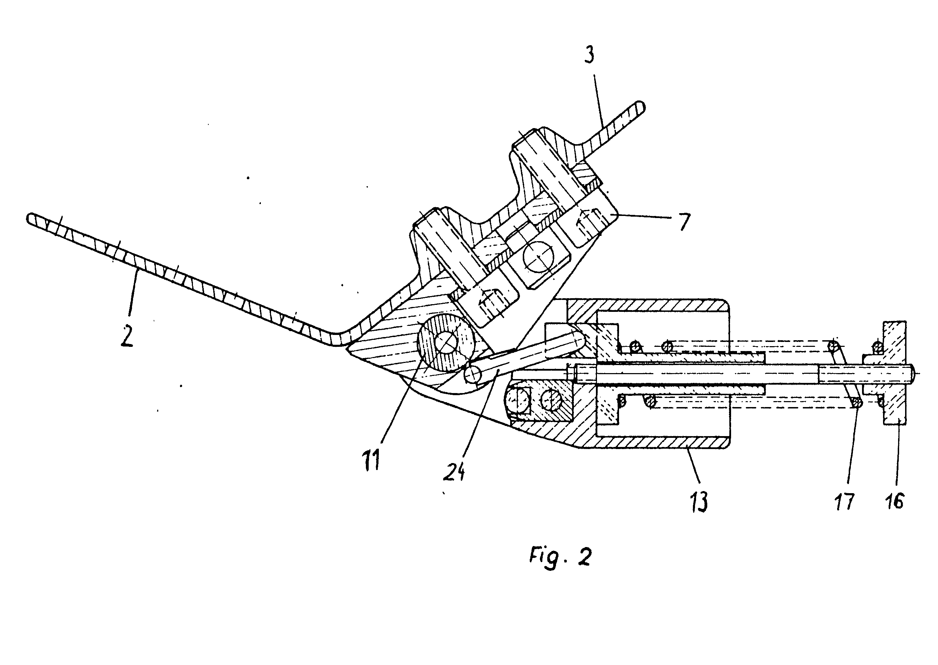

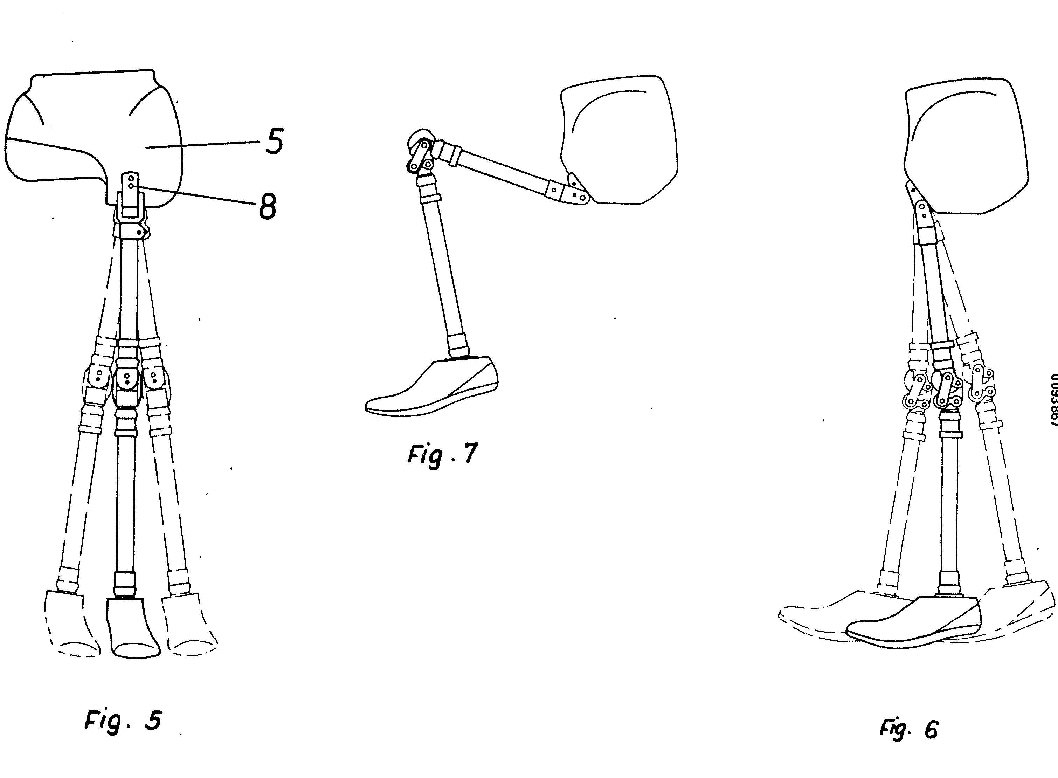

Fig. 1 zeigt einen Befestigungswinkel 1, der einen im wesentlichen horizontal stehenden Schenkel 2 und einen im stumpfen Winkel dazu stehenden aufrechten Schenkel 3 aufweist. Der horizontale Schenkel 2 weist fünf Durchgangslöcher 4 auf, durch die Befestigungsschrauben zur Anbringung an einen in den Fig. 5 bis 7 erkennbaren Befestigungsteil 5 dienen. Statt der Befestigungsschrauben können auch Nieten oder kunstharzgetränkte Laminatfasern Verwendung finden.

Das erfindungsgemässe Hüftgelenk ist mit einem ersten Gelenkteil 6 an dem aufrechten Schenkel 3 des Befestigungswinkels mittels zweier Schrauben 7 angeschraubt. Für den Durchtritt der Schrauben 7 weist das erste Gelenkteil 6 zwei Löcher 8 auf, von denen das obere als kreisbogenförmiges Langloch ausgebildet ist, wie Fig. 4 zeigt. Oberhalb der Löcher 8 befindet sich eine Unterlegplatte 9.

An dem ersten Gelenkteil 6 ist ein zweites Gelenkteil 10 über ein Drehgelenk 11 in der Zeichenebene der Fig. 1 drehbar gelagert. Hierzu umfassen zwei gabelförmige Enden 12 beidseitig das erste Gelenkteil 6. Das zweite Gelenkteil 10 ist zum freien Ende hin als Hohlrohr 13 ausgebildet. Zentrisch in dem Hohlrohr 13 ist eine Achse 14 angeordnet, die am unteren Ende ein Gewinde 15 trägt. Auf das Gewinde 15 ist eine mit einem Innengewinde versehene Einstellscheibe 16 aufgeschraubt. Auf der Einstellscheibe 16 stützt sich eine Druckfeder 17 ab, deren anderes Ende an einer Anschlagscheibe 18 einer relativ zur Achse 14 verschiebbaren Hülse 19 anliegt. Die Hülse 19 ist innerhalb der Schrauben-Druckfeder 17 angeordnet.

Die Anschlagscheibe 18 liegt in der stehenden Stellung des Hüftgelenks an einem oberen Boden 20 des Hohlrohrs 13 an. Durch eine Öffnung 21 in dem Boden 20 ragt nach oben ein Ansatz 22 der Anschlagscheibe 18 hindurch. Der Ansatz 22 ist nach oben hin gabelförmig ausgebildet und weist eine Aufnahmemulde 23 auf, in der sich eine konvexe Endfläche eines Stifts 24 abstützt. Das andere Ende des Stifts 24 umfasst in Form einer Klaue einen parallel zum Drehgelenk 11 ausgerichteten Lagerbolzen 25. Der Stift 24 ist somit um den Lagerbolzen 25 des ersten Drehgelenks 6 und in der Aufnahmemulde 23 des zweiten Drehgelenks 10 drehbar gelagert.

Beim Beugen des Hüftgelenks aus der stehenden Stellung der Fig. 1 nach vorn drückt der Stift 24 den Ansatz 22 und damit die Hülse 19 gegen die Kraft der Druckfeder 17 nach unten. Die Gegenkraft gegen die Beugebewegung des Hüftgelenks nimmt aufgrund der weiteren Kompression der Druckfeder 17 zu, bis der Stift 24 und die Hülse 19 etwa parallel zueinander stehen, d. h. nach einer Beugebewegung von ca. 90° gegenüber der stehenden Stellung aus Fig. 1. Bei einem weiteren Beugen des Hüftgelenks wandert die Hülse 19 aufgrund der Kraft der Druckfeder 17 wieder um ein Stück nach oben, so dass die Feder 17 die weitere Beugebewegung bis in die in Fig. 2 dargestellte sitzende Stellung unterstützt.

Aus Fig. 2 ist erkennbar, dass sowohl die untere Kante 26 des ersten Gelenkteils 6 als auch der obere Teil der hinteren Kante 27 des zweiten Gelenkteils 10 abgeschrägt ausgebildet sind, damit diese Kanten nicht wesentlich nach unten über die gedachte Verlängerungslinie des im wesentlichen horizontalen Schenkels 2 hinausragen.

Das zweite Gelenkteil 10 liegt, wie Fig. 1 erkennen lässt, in der stehenden Stellung mit seiner Oberseite an der unteren Kante 26 des ersten Gelenkteils 6 an. Zur Vermeidung eines störenden Gehgeräusches weist die Oberseite einen Einsatz 28 aus einem elastischen, dämpfenden Material auf. Dieser Einsatz ist höhenverstellbar mit einer Mimik angeordnet, die in Fig. 3 im Detail dargestellt ist. Zur Höhenverstellung ist ein keilförmiges Anschlagstück 29 mit seiner schrägen Fläche auf einer schrägen Fläche eines keilförmigen Gegenstücks 30 gelagert. Eine in dem Gehäuse 31 des zweiten Gelenkteils 10 horizontal unverschiebbar gelagerte Verstellschraube 32 ragt durch eine Durchgangsöffnung 33 des keilförmigen Anschlagteils 29 hindurch und wirkt mit einem Innengewinde 34 in dem keilförmigen Gegenstück 30 zusammen. Durch Anziehen der Schraube 32 wandert das keilförmige Gegenstück 30 in der Darstellung der Fig. 3 nach rechts, wodurch das keilförmige Anschlagstück 29 nach oben verschoben wird, wie dies in Fig. 3 gestrichelt dargestellt ist.

In der sitzenden Stellung des Hüftgelenks liegt ein Bodenteil 35 der gabelförmigen Anordnung 12 des zweiten Gelenkteils 10 an der im wesentlichen vertikal stehenden Kante des ersten Gelenkteils 6 an. Dieser Anschlag bedarf keiner Verstellmöglichkeit.

Die Fig. 5 und 6 zeigen die Verstellmöglichkeit des erfindungsgemässen Hüftgelenks in der Frontalebene und in der Sagittalebene. Die Verstellung in der Frontalebene erfolgt mit Hilfe einer Schraube 36 bei gelösten Schrauben 7 und des kreisbogenförmigen Langlochs 8, während die Verstellung in der Sagittalebene (Fig. 6) mit Hilfe des in Fig. 3 detailliert dargestellten Anschlagstücks erfolgt.

Fig. 7 verdeutlicht, dass das erfindungsgemässe Hüftgelenk in der sitzenden Stellung nach unten hin nicht aufträgt, so dass der Träger des künstlichen Beins in der sitzenden Stellung durch das Hüftgelenk nicht gestört wird.

**Patentansprüche**

1. Hüftgelenk für ein künstliches Bein mit einem aus einem im wesentlichen horizontalen Schenkel (2) und einem im stumpfen Winkel dazu stehenden aufrechten Schenkel (3) bestehenden Befestigungswinkel (1), an dem ein erstes Gelenkteil (6) befestigt ist, das über ein Drehgelenk (11) mit einem zweiten, zur Verbindung mit dem künstlichen Bein vorgesehenen Gelenkteil (10) verbunden ist, wobei die Drehbewegung des zweiten Gelenkteils (10) durch Anschläge (26, 28; 35) für die stehende und die sitzende Stellung des künstlichen Beins beschränkt ist und zwischen dem ersten Gelenkteil (6) und dem zweiten Gelenkteil (10) elastische Mittel (17) eingesetzt sind, die für die Bewegung des zweiten Gelenkteils (10) aus der stehenden in die sitzende Stellung und umgekehrt eine elastische Gegenkraft erzeugen, dadurch gekennzeichnet, dass das erste Gelenkteil (6) am aufrechten Schenkel (3) des Befestigungswinkels (1) angbracht ist und dass das erste Gelenkteil (6) und das zweite Gelenkteil (10) in der sitzenden Stellung nicht oder höchstens unwesentlich über die Verlängerungslinie des im wesentlichen horizontalen Schenkels (2) nach unten herausragen.

2. Hüftgelenk nach Anspruch 1, dadurch gekennzeichnet, dass das erste Gelenkteil (6) um einen kleinen Winkel in vertikaler Ebene drehbar an dem Befestigungswinkel (1) angebracht ist.

3. Hüftgelenk nach Anspruch 2, dadurch gekennzeichnet, dass das erste Gelenkteil (6) zwei untereinander angeordnete Löcher (8) aufweist, durch die Befestigungsschrauben (7) zur Befestigung an dem Befestigungswinkel (1) ragen und von denen mindestens eines als kreisbogenförmiges Langloch ausgebildet ist.

4. Hüftgelenk nach einem der Ansprüche 1 bis 3, dadurch gekennzeichnet, dass das zweite Gelenkteil (10) in der stehenden Stellung mit seiner Oberseite (28) gegen die Unterseite (26) des ersten Gelenkteils (6) stösst und dass die Höhe der Oberseite (28) des zweiten Gelenkteils (10) einstellbar ist.

5. Hüftgelenk nach Anspruch 4, dadurch gekennzeichnet, dass die Oberseite des zweiten Gelenkteils (10) durch ein keilförmiges Anschlagstück (29) gebildet ist, das auf einer schrägen Fläche eines keilförmigen Gegenstücks (30) aufliegt und dass die relative horizontale Lage der beiden keilförmigen Stücke (29, 30) einstellbar ist.

6. Hüftgelenk nach Anspruch 5, dadurch gekennzeichnet, dass eines der keilförmigen Stücke (29, 30) in horizontaler Richtung ein Durchgangsloch (33) aufweist, durch das eine in einem Gewindeloch (34) des anderen keilförmigen Stücks (30, 29) geführte Schraube (32) hindurchragt, die im Gehäuse (31) des Gelenks in horizontaler Richtung unverschiebbar gelagert ist.

7. Hüftgelenk nach einem der Ansprüche 4 bis 6, dadurch gekennzeichnet, dass die Oberseite (28) des zweiten Gelenkteils (10) aus einem elastischen, dämpfenden Material gebildet ist.

8. Hüftgelenk nach einem der Ansprüche 1 bis 7, gekennzeichnet durch einen drehbar am ersten Gelenkteil (6) gelagerten Stift (24), dessen anderes Ende drehbar mit einem Ende einer in dem zweiten Gelenkteil (10) in einem rohrförmigen Ansatz (13) für ein Rohrskeletteil des künstlichen Beins gelagerten Druckfeder (17) verbunden ist.

9. Hüftgelenk nach Anspruch 8, dadurch gekennzeichnet, dass die drehbare Anlenkung des Stifts (24) an dem ersten Gelenkteil (6) oberhalb des Anlenkpunktes an der Druckfeder (17) in der stehenden Stellung und unterhalb des Anlenkpunktes der Druckfeder (17) in der sitzenden Stellung angeordnet ist.

10. Hüftgelenk nach Anspruch 9, dadurch gekennzeichnet, dass die Bewegung des zweiten Gelenkteils (10) einen Winkel von deutlich mehr als 90° umfasst und dass der maximale Federdruck etwa bei einem Winkel von 90° gegenüber der stehenden Stellung auftritt.

11. Hüftgelenk nach einem der Ansprüche 8 bis 10, dadurch gekennzeichnet, dass die Vorspannung der Druckfeder (17) veränderbar ist.

**Claims**

1. Hip-joint for an artificial leg, comprising a fastening angle (1) including a substantially horizontal shank (2) and an upright shank (3) extended to an obtuse angle to said horizontal shank; a first joint member (6) fixed to said fastening angle (1) and connected to a second joint member (10) connectable to the artificial leg by a hinge joint (11), wherein the pivotal movement of the second joint member (10) ist limited by stop means (26, 28; 35) for the standing and for the sitting position of the artificial leg and wherein elastic means (17) are inserted between the first joint member (6) and the second joint member (10), which elastic means generate an elastic counter force for the movement of the second joint member (10) from the standing into the sitting position and vice versa, characterized in that the first joint member (6) is connected to the upright shank (3) of the fastening angle (1) and the first joint member (6) and the second joint member (10) do not or not substantially at a maximum extend downwardly over an extension line of the substantially horizontal shank (2) when the joint is in the sitting position.

2. Hip-joint according to claim 1, characterized in that the first joint member (6) is so connected to the fastening angle (1) that it is pivotable for a small angle in a vertical plane.

3. Hip-joint according to claim 2, characterized in that said first joint member (6) includes two holes (8) positioned below each other, and fastening screws (7) extended through said holes for the connection to the fastening angle (1), at least one of said openings being a circular oblong opening.

4. Hip-joint according to one of claims 1 to 3, characterized in that said second joint member (10) presses its upper side (28) against the lower side (26) of the first joint member (6) in the standing position, the height of the upper side (28) of the second joint member (10) being adjustable.

5. Hip-joint according to claim 4, characterized in that the upper side of the second joint member (10) is formed by a wedge-like support element (29) being supported on an inclined surface of a wedge-like counter element (30), the relative horizontal position of both wedge-like elements (29, 30) being adjustable.

6. Hip-joint according to claim 5, characterized in that one of said wedge-like members (29, 30) includes a through opening (33) in horizontal direction and that a screw (32) which is guided in a threaded opening (34) of the other wedge-like member (30, 29) extends through said opening (33) and is fixed in horizontal direction in the housing (31) of the joint.

7. Hip-joint according to one of the claims 4 to 6, characterized in that the upper side (28) of the second joint member (10) is from an elastic damping material.

8. Hip-joint according to one of claims 1 to 7, characterized by a pin (24) pivotally mounted to said first joint member (6), the other end of said pin (24) being pivotally connected to one end of a compression spring (17) mounted in the second joint member (10) in a tubular projection (13) for a tubular skeleton part of the artificial leg.

9. Hip-joint according to claim 8, characterized in that the pivot of the pin (24) with the first joint member (6) is positioned above the pivot of the pin (24) with the compression spring (17) in the standing position and below the pivot of the pin (24) with the compression spring (17) in the sitting position.

10. Hip-joint according to claim 9, characterized in that the movement of the second joint member (10) covers an angle of substantially more than 90° and that the maximum spring compression occurs approximately at an angle of 90° with respect to the standing position.

11. Hip-joint according to one of the claims 8 to 10, characterized in that the bias of the compression spring (17) is adjustable.

**Revendications**

1. Articulation coxofémorale pour jambe artificielle, comportant une cornière de fixation (1) constituée d'une aile sensiblement horizontale (2) et d'une aile relevée (3) faisant un angle obtus avec la précédente, cornière à laquelle est fixée une première partie (6) de l'articulation, qui est reliée par l'intermédiaire d'un joint pivotant (11) à une deuxième partie (10) prévue pour être reliée à la jambe artificielle, tandis que des butées (26, 28; 35) limitent le mouvement tournant de la deuxième partie (10) de l'articulation en position debout et en position assise de la jambe artificielle, et des moyens élastiques (17) sont interposés entre la première partie (6) et la deuxième partie (10) de l'articulation et produisent une force élastique opposée au déplacement de la deuxième partie (10) de l'articulation de la position debout vers la position assise, et inversement, caractérisé en ce que la première partie (6) de l'articulation est fixée à l'aile relevée (3) de la cornière de fixation (1) et en ce que la première partie (6) et la deuxième partie (10) de l'articulation ne dépassent pas vers le bas, ou ne dépassent au plus que de façon insignifiante vers le bas, la ligne prolongeant l'aile (2) sensiblement horizontale.

2. Articulation coxofémorale selon la revendication 1, caractérisé en ce que la première partie (6) l'articualation est montée sur la cornière de fixation (1) avec une possibilité de rotation d'un petit angle dans le plan vertical.

3. Articulation coxofémorale selon la revendication 2, caractérisé en ce que la première partie (6) de l'articulation présente deux trous (8) disposés l'un en dessous de l'autre, qui sont traversés par des vis de fixation (7) pour la fixation à la cornière de fixation (1) et dont l'un au moins a la forme d'un trou allongé selon un arc de cercle.

4. Articulation coxofémorale selon l'une des revendications 1 à 3, caractérisé en ce que la deuxième partie (10) de l'articulation bute, en position debout, par sa face supérieure (28) contre la face inférieure (26) de la première partie (6) de l'articulation, et en ce que la hauteur de la face supérieure (28) de la deuxième partie (10) de l'articulation est réglable.

5. Articulation coxofémorale selon la revendication 4, caractérisé en ce que la face supérieure de la deuxième partie (10) de l'articulation est constituée par une pièce de butée en forme de coin (29), qui repose sur une face inclinée d'une contrepièce en forme de coin (30) et en ce que la position horizontale relative des deux pièces en forme de coin (29, 30) est réglable.

6. Articulation coxofémorale selon la revendication 5, caractérisée en ce que l'une des pièces en forme de coin (29, 30) présente en direction horizontale un trou traversant (33), à travers lequel passe une vis (32) guidée dans un trou taraudé (34) de l'autre pièce en forme de coin (30, 29), ladite vis étant positionnée de manière horizontalement indéplaçable dans le boîtier (31) de l'articulation.

7. Articulation coxofémorale selon l'une des revendications 4 à 6, caractérisée en ce que la face supérieure (28) de la deuxième partie (10) de l'articulation, est formée par un matériau élastique et amortisseur.

8. Articulation coxofémorale selon l'une des revendications 1 à 7, caractérisée en ce qu'une biellette (24) montée rotativement dans la première partie (6) de l'articulation, est reliée rotativement par son autre extrémité avec l'une des extrémités d'un ressort de compression (17) positionnée, dans la deuxième partie (10) de l'articulation, dans un appendice tubulaire (13) pour un squelette tubulaire de la jambe artificielle.

9. Articulation coxofémorale selon la revendication 8, caractérisée en ce que l'articulation rotative de la biellette (24) sur la première partie (6) de l'articulation est positionnée au-dessus du point d'articulation avec le ressort de compression (17) dans la position debout et en dessous du point d'articulation avec le ressort de compression (17) dans la position assise.

10. Articulation coxofémorale selon la revendication 9, caractérisée en ce que l'angle de déplacement de la deuxième partie (10) de l'articulation est significativement supérieur à 90° et en ce que la pression élastique du ressort est maximale lorsque l'angle est d'environ 90° par rapport à la position debout.

11. Articulation coxofémorale selon l'une des revendications 8 à 10, caractérisée en ce que la contrainte initiale du ressort de compression (17) est réglable.

0093867

Fig. 4

Fig. 3

Fig. 1

Fig. 2

Fig. 5

Fig. 7

Fig. 6

0093867